# EUROPEAN PATENT APPLICATION

(11) **EP 2 644 706 A2**
(43) Date of publication of application: **02.10.2013**
(21) Application number: 13166434.4
(22) Date of filing: 25.06.2010
(51) Int. Cl.: C12Q 1/68

(54) **Method for evaluation of cultured cells, and method for screening of biomarker**

(30) Priority: 30.06.2009 JP 2009154449
(62) Divisional of application: 10794075.1
(71) Applicant: FUJIREBIO INC., Shinjuku-ku, Tokyo 163-0410 (JP)
(72) Inventor: Hotta, Yoshiyuki, Tokyo 103-0007 (JP); Omi, Kazuya, Tokyo 103-0007 (JP); Oka, Asako, Tokyo 103-0007 (JP)
(74) Representative: Keirstead, Tanis Evelyne

(57) **Abstract**

Disclosed are novel means by which evaluation of cultured cells and screening of biomarkers can be attained without consuming the cultured cells. A method for evaluating cultured cells according to the present invention comprises culturing cells in a serum-free medium, and measuring at least one nucleic acid released from the cells into the culture medium. A method for screening of a biomarker according to the present invention comprises culturing cells in a serum-free medium, and measuring a nucleic acid(s) released from the cells into the culture medium. Nucleic acid used as an indicator is e.g. microRNA.

SEQUENCE LISTING

<110>  FUJIREBIO INC.

<120>  Method for evaluation of cultured cells, and method for screening of
biomarker

<130>  TEK/FP6897235

<140>  EP
<141>  2010-06-25

<150>  EP10794075.1
<151>  2010-06-25

<150>  PCT/JP2010/060812
<151>  2010-06-25

<150>  JP2009-154449
<151>  2009-06-30

<160>  44

<170>  PatentIn version 3.1

<210>  1
<211>  23
<212>  RNA
<213>  Homo sapiens

<400>  1
guccaguuuu cccaggaauc ccu                                              23


<210>  2
<211>  22
<212>  RNA
<213>  Homo sapiens

<400>  2
uggcucaguu cagcaggaac ag                                               22


<210>  3
<211>  22
<212>  RNA
<213>  Homo sapiens

<400>  3
uagcagcacg uaaauauugg cg                                               22


<210>  4
<211>  22
<212>  RNA
<213>  Homo sapiens

<400>  4
uagcuuauca gacugauguu ga                                               22


<210>  5
<211>  22

<212>  RNA
<213>  Homo sapiens

<400>  5
uggaguguga caaugguguu ug                                              22


<210>  6
<211>  22
<212>  RNA
<213>  Homo sapiens

<400>  6
aaaccguuac cauuacugag uu                                              22


<210>  7
<211>  23
<212>  RNA
<213>  Homo sapiens

<400>  7
uaaagugcuu auagugcagg uag                                             23


<210>  8
<211>  21
<212>  RNA
<213>  Homo sapiens

<400>  8
   cacugugucc uuucugcgua g                                              21


<210>  9
<211>  22
<212>  RNA
<213>  Homo sapiens

<400>  9
aguucuucag uggcaagcuu ua                                              22


<210>  10
<211>  23
<212>  RNA
<213>  Homo sapiens

<400>  10
ugugcaaauc uaugcaaaac uga                                             23


<210>  11
<211>  22
<212>  RNA
<213>  Homo sapiens

<400>  11
ucaggcucag uccccucccg au                                              22


<210>  12
<211>  25

<212>  RNA
<213>  Homo sapiens

<400>  12
agggaucgcg ggcggguggc ggccu                                            25


<210>  13
<211>  22
<212>  RNA
<213>  Homo sapiens

<400>  13
ucccugagac ccuaacuugu ga                                               22


<210>  14
<211>  23
<212>  RNA
<213>  Homo sapiens

<400>  14
ucccuguccu ccaggagcuc acg                                              23


<210>  15
<211>  22
<212>  RNA
<213>  Homo sapiens

<400>  15
ccucccacac ccaaggcuug ca                                               22


<210>  16
<211>  23
<212>  RNA
<213>  Homo sapiens

<400>  16
uguaguguuu ccuacuuuau gga                                              23


<210>  17
<211>  23
<212>  RNA
<213>  Homo sapiens

<400>  17
agcugguguu gugaaucagg ccg                                              23


<210>  18
<211>  23
<212>  RNA
<213>  Homo sapiens

<400>  18
caaagaauuc uccuuuuggg cuu                                              23


<210>  19
<211>  21

<212>  RNA
<213>  Homo sapiens

<400>  19
ugucaguuug ucaaauaccc c                                                21


<210>  20
<211>  46
<212>  DNA
<213>  Artificial

<220>
<223>  RT primer

<220>
<221>  misc_feature
<222>  (45)..(45)
<223>  v is not t


<220>
<221>  misc_feature
<222>  (46)..(46)
<223>  n is any base


<400>  20
gcgagcacag aattaatacg actcactata ggtttttttt ttttvn                     46


<210>  21
<211>  22
<212>  DNA
<213>  Artificial

<220>
<223>  reverse primer

<400>  21
gcgagcacag aattaatacg ac                                               22


<210>  22
<211>  22
<212>  DNA
<213>  Artificial

<220>
<223>  primer for hsa-miR-145

<400>  22
ccgcgtccag ttttcccagg aa                                               22


<210>  23
<211>  20
<212>  DNA
<213>  Artificial

<220>
<223>  primer for hsa-miR-24

<400>  23
ccgctggctc agttcagcag                                                    20


<210>  24
<211>  20
<212>  DNA
<213>  Artificial

<220>
<223>  primer for hsa-miR-16

<400>  24
cgcgctagca gcacgtaaat                                                    20


<210>  25
<211>  24
<212>  DNA
<213>  Artificial

<220>
<223>  primer for hsa-miR-21

<400>  25
gcccgctagc ttatcagact gatg                                               24


<210>  26
<211>  21
<212>  DNA
<213>  Artificial

<220>
<223>  primer for hsa-miR-122

<400>  26
gcgctggagt gtgacaatgg t                                                  21


<210>  27
<211>  22
<212>  DNA
<213>  Artificial

<220>
<223>  primer for hsa-miR-451

<400>  27
gccgcaaacc gttaccatta ct                                                 22


<210>  28
<211>  23
<212>  DNA
<213>  Artificial

<220>
<223>  primer for hsa-miR-20a

<400>  28
ccgccgctaa agtgcttata gtg                                                23

<210>  29
<211>  21
<212>  DNA
<213>  Artificial

<220>
<223>  primer for hsa-miR-892a

<400>  29
ccgccactgt gtcctttctg c                                                21


<210>  30
<211>  21
<212>  DNA
<213>  Artificial

<220>
<223>  primer for hsa-miR-22*

<400>  30
ccgcgagttc ttcagtggca a                                                21


<210>  31
<211>  21
<212>  DNA
<213>  Artificial

<220>
<223>  primer for hsa-miR-19a

<400>  31
ccgcctgtgc aaatctatgc a                                                21


<210>  32
<211>  19
<212>  DNA
<213>  Artificial

<220>
<223>  primer for hsa-miR-484

<400>  32
ccgtcaggct cagtcccct                                                   19


<210>  33
<211>  17
<212>  DNA
<213>  Artificial

<220>
<223>  primer for hsa-miR-638

<400>  33
ccgcagggat cgcgggc                                                     17


<210>  34
<211>  20

<212>  DNA
<213>  Artificial

<220>
<223>  primer for hsa-miR-125b

<400>  34
ccgcgtccct gagaccctaa                                                    20


<210>  35
<211>  19
<212>  DNA
<213>  Artificial

<220>
<223>  primer for hsa-miR-339-5p

<400>  35
ccgtccctgt cctccagga                                                     19


<210>  36
<211>  19
<212>  DNA
<213>  Artificial

<220>
<223>  primer for hsa-miR-532-3p

<400>  36
ccctcccaca cccaaggct                                                     19


<210>  37
<211>  23
<212>  DNA
<213>  Artificial

<220>
<223>  primer for hsa-miR-142-3p

<400>  37
ccgcctgtag tgtttcctac ttt                                                23


<210>  38
<211>  20
<212>  DNA
<213>  Artificial

<220>
<223>  primer for hsa-miR-138

<400>  38
ccggcagctg gtgttgtgaa                                                    20


<210>  39
<211>  23
<212>  DNA
<213>  Artificial

<220>
<223>  primer for hsa-miR-186

<400>  39
ccgccgcaaa gaattctcct ttt                                              23


<210>  40
<211>  23
<212>  DNA
<213>  Artificial

<220>
<223>  primer for hsa-miR-223

<400>  40
ccgccgtgtc agtttgtcaa ata                                              23


<210>  41
<211>  22
<212>  RNA
<213>  Homo sapiens

<400>  41
cagugcaaug uuaaaagggc au                                               22


<210>  42
<211>  21
<212>  RNA
<213>  Homo sapiens

<400>  42
ugagaugaag cacuguagcu c                                                21


<210>  43
<211>  22
<212>  RNA
<213>  Homo sapiens

<400>  43
acagcaggca cagacaggca gu                                               22


<210>  44
<211>  22
<212>  RNA
<213>  Homo sapiens

<400>  44
uaaugccccu aaaaauccuu au                                               22

## Description

### TECHNICAL FIELD

The present invention relates to a method for evaluating cultured cells and a method for screening of a biomarker(s), which are carried out using as an indicator a nucleic acid(s) such as a microRNA(s) (miRNA(s)) released from cultured cells into a culture medium.

### BACKGROUND ART

Evaluation of cultured cells is carried out by immunostaining of the cultured cells or analysis of proteins or genes expressed in the cells. For example, embryonic stem cells are evaluated by immunostaining of OCT4, NANOG and the like; and cells obtained by inducing differentiation of embryonic stem cells are evaluated based on decrease in expression level of OCT4 and NANOG genes or increase in expression level of tissue-specific genes.

When evaluation methods in which cells are used such as immunostaining and gene expression measurement are carried out, cultured cells have to be fixed or lysed. Therefore, for the purpose of evaluating changes with time, a large amount of cultured cells have to be prepared under the same culture conditions. It is also problematic that the cultured cells must be consumed with time.

In the field of regenerative medicine (cell therapy), which is attracting attention as a new method of treatment, there is a problem that cells to be transplanted have to be evaluated in an indirect manner, for example, by staining of the cells cultured under the same conditions. It is impossible by any conventional method to evaluate the exact cells to be transplanted.

Recent interest has focused on miRNA, a single-stranded RNA which is generated by degradation of hairpin-folded RNA of about 60-100 bases (precursor microRNA) transcribed from a miRNA-encoding gene by ribonuclease into a size of about 17-24 bases. The miRNA molecules have a function to inhibit translation of messenger RNA comprising the base sequence complementary to themselves, and have been reported to play an important role in differentiation of stem cells into tissue cells (Non-patent Document 1) and determination of cancer cells of various tissues (Patent Document 1). It has been also reported that miRNA is present in blood (Non-patent Document 2), which draws attention to the possibility that miRNA may be useful for diagnosis, prognostication etc. of diseases such as cancers.

A medium supplemented with serum is generally used in culturing cells from the viewpoint of increasing the culture efficiency. Since it is difficult to distinguish between nucleic acids which come from serum added to the medium and nucleic acids released from the cultured cells, there are no reports on a method for evaluating cells nor a method for screening of biomarkers using as an indicator nucleic acids released from cultured cells.

### PRIOR ART DOCUMENTS

### PATENT DOCUMENTS

Patent Document 1: JP 2009-100687 A

### NON-PATENT DOCUMENTS

Non-patent Document 1: Exp. Hematol. 2008;36:1354-1369
Non-patent Document 2: Proc. Natl. Acad. Sci. USA. 2008; 105:10513-8

### SUMMARY OF THE INVENTION

### PROBLEMS TO BE SOLVED BY THE INVENTION

Accordingly, an object of the present invention is to provide novel means by which evaluation of culture cells or screening of biomarkers can be carried out without consuming culture cells.

### MEANS FOR SOLVING THE PROBLEMS

The present inventors intensively studied to find that nucleic acids such as miRNAs are released from cultured cells into a culture medium, and that the amount and the kind of the nucleic acids such as miRNA released into a culture medium vary depending on stem cells, differentiated tissue cells, drug treatment, malignancy development of cancer cells, and so on. And thus they found that the state of cells can be evaluated by usnig the nucleic acids such as miRNAs as an indicator, thereby completing the present invention. In addition, the inventors found that biomarkers can be screened by measuring the above-mentioned nucleic acids such as miRNAs in a serum-free medium, thereby completing the present invention.

That is, the present invention provides a method for evaluating cultured cells, comprising culturing cells in a serum-free medium, and measuring at least one nucleic acid released from the cells into the culture medium. In this method, cells are cultured in a serum-free medium, and therefore the present invention provides a screening method by which a nucleic acid which is a candidate of a biomarker for stem cells, cancer cells and so on can be easily found.

More specifically, the present invention provides the following inventions.
(1) A method for evaluating cultured cells, comprising culturing cells in a serum-free medium, and measuring at least one nucleic acid released from the cells into the culture medium.
(2) The method according to (1), wherein said nucleic acid is microRNA.
(3) The method according to (2), wherein at least one of the microRNAs composed of the base sequences shown in SEQ ID NOs: 1, 3-5, 7-19, 41-44 in SEQUENCE LISTING is measured.
(4) The method according to (3), wherein at least one of the microRNAs composed of the base sequences shown in SEQ ID NOs: 1, 3-5, 7-19 in SEQUENCE LISTING is measured.
(5) The method according to any one of (1) to (4), wherein said cells are originated from a mammal, and wherein said serum-free medium contains a ligand for an endothelial cell differentiation gene (Edg) family receptor and a ligand for a serotonin receptor.
(6) The method according to (5), wherein said ligand for an endothelial cell differentiation gene family receptor is at least one selected from the group consisting of: lysophosphatidic acid (LPA) and salts thereof; sphingosine-1-phosphate (S1P); and agonists of endothelial cell differentiation gene (Edg) family receptors.
(7) The method according to (5) or (6), wherein said ligand for a serotonin receptor is at least one selected from the group consisting of: serotonin and salts thereof; and agonists of serotonin receptors.
(8) The method according to any one of (1) to (7), which is a method for evaluating differentiation of stem cells.
(9) The method according to (8), wherein differentiation of stem cells is evaluated using at least one of the microRNAs composed of the base sequences shown in SEQ ID NOs: 1, 41-44 as an indicator.
(10) The method according to (9), wherein differentiation into osteocytes is evaluated using a microRNA composed of the base sequence shown in SEQ ID NO: 1 or 44 as an indicator.
(11) The method according to (10), wherein differentiation into osteocytes is evaluated using a microRNA composed of the base sequence shown in SEQ ID NO: 1 as an indicator.
(12) The method according to (9), wherein differentiation into adipocytes is evaluated using a microRNA composed of the base sequence shown in SEQ ID NO: 41 as an indicator.
(13) The method according to (9), wherein differentiation into tissue cells is evaluated using a microRNA composed of the base sequence shown in SEQ ID NO: 42 as an indicator.
(14) The method according to (9), wherein whether differentiation of stem cells occurs is evaluated using a microRNA composed of the base sequence shown in SEQ ID NO: 43 as an indicator.
(15) The method according to any one of (1) to (7), which is a method for evaluating cell injury.
(16) The method according to (15), wherein injury to the cultured cells is evaluated using at least one of the microRNAs composed of the base sequences shown in SEQ ID NOs: 3-5 as an indicator.
(17) The method according to (16), wherein said cultured cells are liver cells.
(18) The method according to any one of (1) to (7), which is a method for evaluating effect, influence, toxicity, etc. of a chemical substance, biological material, environmental stimulus, etc. against cultured cells.
(19) The method according to any one of (1) to (7), which is a method for evaluating the presence of cancer cells.
(20) The method according to any one of (1) to (7), which is a method for evaluating malignancy of cancer cells.
(21) The method according to (19) or (20), wherein at least one of the microRNAs composed of the base sequences shown in SEQ ID NOs: 7-19 is used as an indicator.
(22) The method according to (21), wherein said cancer cells are colon cancer cells.
(23) A method for screening a biomarker(s), comprising culturing cells in a serum-free medium, and measuring a nucleic acid(s) released from the cells into the culture medium.
(24) The method according to (23), wherein at least one of the microRNAs composed of the base sequences shown in SEQ ID NOs: 1, 3-5, 7-19, 41-44 in SEQUENCE LISTING is measured.
(25) The method according to (24), wherein at least one of the microRNAs composed of the base sequences shown in SEQ ID NOs: 1, 3-5, 7-19 in SEQUENCE LISTING is measured.

### EFFECTS OF THE INVENTION

By the present invention, novel methods for cell evaluation and biomarker screening in which a nucleic acid(s) released into a culture medium is(are) used as an indicator were provided. In the method of the present invention, there is no need to consume cells only for the purpose of an evaluation, which means that there is no need to prepare additional culture cells for the evaluation and that time and money can be largely saved. It is also a great advantage that consumption of a valuable cell sample can be avoided. Moreover, the present invention is also very useful for evaluation of cells in the field of regenerative medicine and the like, since cells which will be actually used can be directly evaluated by the present invention. Furthermore, the present invention is useful for attaining an easy screening of a biomarker(s).

### BRIEF DESCRIPTION OF THE DRAWINGS

Fig. 1 shows the results obtained by examining the pattern of the amount of miRNAs in a culture medium when mesenchymal stem cells were induced to differentiate into osteocytes or adipocytes in Example 1.
Fig. 2 shows the results obtained by examining the pattern of the amount of miRNAs in a culture medium when cultured cells derived from human liver cancer were injured by treatment with carbon tetrachloride in Example 2.
Fig. 3 shows the results obtained by examining the pattern of the relative expression of miRNAs in culture media of human colon cancer-derived cell line SW480 and its lymph node metastasis-derived cell line SW620 in Example 3, which relative expression amount was calculated by correcting the amount of miRNAs by the amount of hsa-miR-16.
Fig. 4 shows the results obtained by examining the pattern of miRNAs in a culture medium when human bone marrow-derived mesenchymal stem cells were induced to differentiate into osteocytes or adipocytes in Example 4.

### MODE FOR CARRYING OUT THE INVENTION

In the present invention, "evaluation of cells" means evaluation of the state of cells, and includes evaluation of various states such as cell differentiation; the effect, influence, toxicity, etc. of chemical substances, biological materials (e.g. nucleic acids, proteins and fragments thereof, carbohydrates, lipids, and vitamins), environmental stimuli (e.g. decrease or increase in temperature, decrease or increase in humidity, decrease or increase in pressure, decrease or increase in oxygen concentration, decrease or increase in CO₂ concentration, ultraviolet irradiation, and radiation), etc.; presence of cancer cells; and malignancy of cancer cells. The term "biomarker" means any material originated from a living body (e.g. nucleic acids, proteins and fragments thereof, carbohydrates, lipids, and vitamins) which may be used as an indicator for qualitatively and/or quantitatively understanding a change in the state of cells, tissues, living bodies and the like.

The nucleic acid to be measured in the present invention may be of any kind as long as it is a nucleic acid released from the cultured cells into a culture medium. The nucleic acid may be DNA or RNA, and preferably microRNA (miRNA). In the present invention, "miRNA" is a single-stranded RNA which is generated by degradation of hairpin-folded RNA of about 60-100 bases transcribed from a miRNA-encoding gene by ribonuclease into a size of about 17-24 bases, and has a function to inhibit translation of messenger RNA comprising the base sequence complementary to itself. Genes encoding miRNA are known, and various kinds of miRNAs are also known.

Specific examples of the miRNA to be measured include miRNAs composed of the base sequences shown in SEQ ID NOs: 1, 3-5, 7-19 in SEQUENCE LISTING, all of which are demonstrated in the following Examples to vary in their amounts in the medium depending on differentiation of cells, cell injury, presence of cancer cells, malignancy development of cancer cells and the like. However, the nucleic acid to be measured is not restricted to these miRNAs.

For example, as shown in the following Examples, miRNAs composed of the base sequences shown in SEQ ID NOs: 1, 41-44 (hsa-miR-145, hsa-miR-130a, hsa-miR-143, hsa-miR-214, hsa-miR-365) may be used as an indicator of cell differentiation. More specifically, miRNAs of SEQ ID NO: 1 and 44 (hsa-miR-145, hsa-miR-365) may be used as an indicator of the differentiation of stem cells into osteocytes; and a miRNA of SEQ ID NO: 41 (hsa-miR-130a) may be used as an indicator of the differentiation of stem cells into adipocytes. A miRNA of SEQ ID NO: 42 (hsa-miR-143) may be used as an indicator of the differentiation into tissue cells including osteocytes and adipocytes, since it is measured in large amounts in both cases where differentiation into osteocytes occurs and where differentiation into adipocytes occurs. A miRNA of SEQ ID NO: 43 (hsa-miR-214) may be used as an indicator of whether differentiation of stem cells occurs, since it is measured in large amounts in a culture medium of undifferentiated stem cells whose differentiation is not yet induced.

miRNAs composed of the base sequences shown in SEQ ID NOs: 3-5 (hsa-miR-16, hsa-miR-21, hsa-miR-122) may be used as an indicator of cell injury including cytotoxicity of a chemical substance etc., more specifically, liver cell injury by drug treatment.

miRNAs composed of the base sequences shown in SEQ ID NOs: 7-19 (hsa-miR-20a, hsa-miR-892a, hsa-miR-22*, hsa-miR-19a, hsa-miR-484, hsa-miR-638, hsa-miR-125b, hsa-miR-339-5p, hsa-miR-532-3p, hsa-miR-142-3p, hsa-miR-138, hsa-miR-186, hsa-miR-223) may be used as an indicator of cancer malignancy, more specifically colon cancer malignancy. Among those composed of SEQ ID NOs: 7-19, miRNAs composed of the base sequences shown in SEQ ID NOs: 7, 10-15 (hsa-miR-20a, hsa-miR-19a, hsa-miR-484, hsa-miR-638, hsa-miR-125b, hsa-miR-339-5p, hsa-miR-532-3p) are detected in common in culture media regardless of the malignancy of cancers, and therefore may be used as an indicator of the presence of cancer cells. miRNAs composed of the base sequences shown in SEQ ID NOs: 8, 9 (hsa-miR-892a, hsa-miR-22*) are detected specifically in culture media of cell lines derived from a primary site, and miRNAs composed of the base sequences shown in SEQ ID NOs: 16-19 (hsa-miR-142-3p, hsa-miR-138, hsa-miR-186, hsa-miR-223) are detected specifically in culture media of cell lines derived from a metastatic focus, whose malignancy is higher.

Any one or a plurality of the above-described miRNAs may be measured. The kind of the nucleic acids to be measured may be appropriately selected depending on the purpose of the evaluation. For example, when an evaluation of the differentiation of stem cells into osteocytes is desired, hsa-miR-214, the indicator of undifferentiated cells, and hsa-miR-143, the indicator of differentiation into tissue cells, may be measured in combination with hsa-miR-145 and hsa-miR-365, the indicators of differentiation into osteocytes.

Cells cultured in the present invention are not restricted as long as they can be cultured under *in vitro* conditions, and are preferably those originated from a mammal. Cells which constitute a tissue excised from a mammal are also included therein, as long as they can be cultured under *in vitro* conditions.

Examples of the cells originated from a mammal which can be cultured *in vitro* include, but not restricted to, stem cells (e.g., embryonic stem cells, induced pluripotent stem cells, mesenchymal stem cells, hematopoietic stem cells, neural stem cells, skin stem cells), tissue progenitor cells and tissue cells (e.g., osteocytes, osteoblasts, adipocytes, chondrocytes, skin cells, neurons, muscle cells, hematopoietic cells, fibroblasts, liver cells), cancer cells and cancer-derived cell lines (e.g., HepG2, HuH-7, SW480, SW620, Caco-2, CH-4, CH-5, CoLo-205, Hc110, PMP-1).

A medium used for culturing cells in the present invention may be so-called "serum-free medium", which is a medium containing no animal serum additive. A medium which contains an additional additive(s) (excluding animal serum) in a known basal medium may be used. The composition of the basal medium may be appropriately selected depending on the kind of cells to be cultured. Examples of the basal medium include minimum essential medium (MEM) such as Eagle's medium; Dulbecco's modified Eagle's medium(DMEM); minimum essential medium α (MEM-α); mesenchymal stem cell basal medium (MSCBM); Ham's F-12 and F-10 media; DMEM/F12 medium; Williams medium E; RPMI-1640 medium; MCDB medium; 199 medium; Fisher medium; Iscove's modified Dulbecco's medium (IMDM); and McCoy modified medium. These media are all well known in the art.

Examples of the additional additive added to a basal medium include amino acids, inorganic salts, vitamins and other additives such as carbon sources and antibiotics. The concentration of use of these additives is not restricted, and they may be used at a concentration used in a conventional medium for mammalian cells.

Examples of the amino acids include glycine, L-alanine, L-arginine, L-asparagine, L-aspartic acid, L-cysteine, L-cystine, L-glutamic acid, L-glutamine, L-histidine, L-isoleucine, L-leucine, L-lysine, L-methionine, L-phenylalanine, L-proline, L-serine, L-threonine, L-tryptophan, L-tyrosine and L-valine.

Examples of the inorganic salts include calcium chloride, copper sulfate, iron (III) nitrate, iron sulfate, magnesium chloride, magnesium sulfate, potassium chloride, sodium hydrogen carbonate, sodium chloride, disodium hydrogen phosphate and sodium dihydrogen phosphate.

Examples of the vitamins include choline, vitamin A, vitamin B1, vitamin B2, vitamin B3, vitamin B4, vitamin B5, vitamin B6, vitamin B7, vitamin B12, vitamin B 13, vitamin B 15, vitamin B 17, vitamin Bh, vitamin Bt, vitamin Bx, vitamin C, vitamin D, vitamin E, vitamin F, vitamin K, vitamin M and vitamin P.

Examples of other additives include (1) growth factors such as fibroblast growth factors (FGFs), endothelial growth factors (EGFs), and platelet-derived growth factors (PDGFs); (2) antibiotics such as penicillin, streptomycin, gentamycin and kanamycin; (3) carbon sources such as glucose, galactose, fructose and sucrose; (4) trace metals such as magnesium, iron, zinc, calcium, potassium, sodium, copper, selenium, cobalt, tin, molybdenum, nickel and silicon; (5) stem cell differentiation inducers such as β-glycerophosphoric acid, dexamethasone, rosiglitazone, isobutylmethylxanthine, and 5-azacytidine; (6) antioxidants such as 2-mercaptoethanol, catalase, superoxide dismutase and N-acetylcysteine; and other additives such as adenosine 5'-monophosphate, corticosterone, ethanolamine, insulin, reduced glutathione, lipoic acid, melatonin, hypoxanthine, phenol red, progesterone, putrescine, pyruvic acid, thymidine, triiodothyronine, transferrin and lactoferrin.

There are various known media and culture methods which make it possible to culture animal cells under serum-free conditions, and any of such media and methods may be used in the present invention. Especially, a medium containing a ligand for an endothelial cell differentiation gene (Edg) family receptor and a ligand for a serotonin receptor (this medium is hereinafter referred to as "efficient serum-free medium" for convenience) may be preferably used as a medium in which mammalian cells can be grown effectively.

As described above, the efficient serum-free medium contains a ligand for an Edg family receptor as one of the essential components. Edg family receptors are a group of G protein-coupled receptors whose gene sequences are highly homologous to each other, and Edg-1 to Edg-8 have been identified so far in mammals such as human, mouse and sheep (Science. Vol. 294, pp. 1875-1878, 2001, and J Biol Chem. Vol. 277, No.29, pp. 25851-25854, 2002). It is known that, among these, Edg-2, Edg-4 and Edg-7 act as a LPA receptor, and Edg-1, Edg-3, Edg-5, Edg-6 and Edg-8 act as a S1P receptor. "A ligand for a receptor" means a substance which binds specifically to the receptor, and include not only natural ligands existing in a living body but also other naturally-occurring and synthesized compounds known as agonists and antagonists.

As the ligand for an Edg family receptor (this may be hereinafter referred to as "Edg ligand" for convenience), one or a plurality of compounds selected from the group consisting of lysophosphatidic acid (LPA) and salts thereof; sphingosine-1-phosphate (S1P); and agonists of Edg family receptors are preferred.

"Agonists of Edg family receptors" means substances which bind to Edg family receptors and act in the same manner as LPA and S1P, and examples thereof include dihydrosphingosine-1-phosphate, platelet-activating factors (PAFs), sphingosine phosphorylcholine, alkyl LPA analogues and FTY720.

LPA is a compound represented by the General Formula (I) below:

R-O-CH₂CH(OH)CH₂PO₄H₂ (I)

(wherein R represents C₁₀-C₃₀ alkyl, C₁₀-C₃₀ alkenyl or C₁₀-C₃₀ acyl).

The number of carbon atoms of acyl group in the group R in the above-described General Formula (I) does not include carbon atoms of carbonyl group.

As the salt of LPA, a known salt may be used, and examples thereof include alkaline metal salts such as sodium salt and potassium salt; and ammonium salt.

Examples of LPA or the salt of LPA include 1-oleoyl lysophosphatidic acid sodium salt and LPA potassium salt.

Edg ligands may be used either individually or in combination of two or more kinds thereof.

The efficient serum-free medium mentioned above further contains a ligand for a serotonin receptor (this may be hereinafter referred to as "serotonin ligand" for convenience). A serotonin receptor is a kind of G protein-binding receptors existing mainly in a central nerve system. As the serotonin ligand, one or a plurality of compounds selected from serotonin, salts thereof and serotonin agonists are preferred. Serotonin is also called 5-hydroxytryptamine and known to act as a neurotransmitter. A serotonin agonist is a substance known to bind to a serotonin receptor and act in the same manner as serotonin, and examples thereof include ipsapirone, gepirone, buspirone, 1-[2-(4-aminophenyl)ethyl]-4-(3-bifluoromethylphenyl)piperazine (PADD), N,N-dipropyl-5-carboxyamidotryptamine (DP-5CT), α-methyl-5-hydroxytryptamine (HT) and 2-methyl-5-HT. As the salt of serotonin, a known salt may be used, and examples thereof include hydrochloride.

Serotonin ligands may be used either individually or in combination of two or more kinds thereof.

The concentration of the ligand in the medium (in cases where a plurality of the ligands are contained, the total concentration thereof) is usually about 0.01 to 100 µM. In cases where the Edg ligand is at least one selected from the group consisting of lysophosphatidic acid (LPA) and salts thereof, its concentration in the medium is preferably 0.25 to 10 µM. Further, in cases where the Edg ligand is sphingosine-1-phosphate (S1P), its concentration in the medium is preferably 0.01 µM to 0.2 µM. Further, the concentration of the serotonin ligand (in cases where a plurality of the ligands are contained, the total concentration thereof) is preferably 0.1 to 100 µM, more preferably 0.25 to 20 µM.

The efficient serum-free medium preferably further contains an antioxidant. Preferred examples of the antioxidant include at least one selected from the group consisting ofN-acetylcysteine (NAC), L-cysteine, catalase, superoxide dismutase and 2-mercaptoethanol, more preferably, at least one selected from the group consisting of N-acetylcysteine and L-cysteine. These antioxidants are known to have an action to inhibit apoptosis and therefore effective for maintenance and growth of cultured cells. The antioxidants may be used either individually or in combination of two or more kinds thereof.

The concentration of the antioxidant in the medium (in cases where a plurality of the antioxidants are contained, the total concentration thereof) is preferably 0.01 mM to 10 mM, more preferably 0.1 mM to 1 mM.

The efficient serum-free medium preferably further contains an animal serum albumin. Albumin is a major component of serum and known to play roles such as deliver of a drug in blood. By containing an animal serum albumin, growth of cultured cells is further promoted. Preferred examples of the animal serum albumin include human serum albumin (HSA) and recombinant human serum albumin (rHSA), and bovine serum albumin (BSA). These albumins may be used either individually or in combination of two or more kinds thereof.

The concentration of albumin in the medium (in cases where a plurality of the albumins are contained, the total concentration thereof) is preferably 0.0001 to 10% by weight, more preferably 0.0001 to 1% by weight.

The efficient serum-free medium preferably further contains a growth factor. By containing a growth factor, growth of cultured cells is further promoted. Preferred examples of the growth factor include epidermal growth factors (EGFs), insulin-like growth factors (IGFs), transforming growth factors (TGFs), nerve growth factors (NGFs), brain-derived neurotrophic factors (BDNFs), vascular endothelial cell growth factors (VEGFs), granulocyte colony-stimulating factors (G-CSFs), granulocyte-macrophage colony-stimulating factors (GM-CSFs), erythropoietin (EPO), thrombopoietin (TPO) and hepatocyte growth factors (HGFs), and more preferred examples thereof include platelet-derived growth factors (PDGFs), basic fibroblast growth factors (bFGFs) and epidermal growth factors (EGFs). These growth factors themselves are well-known in the field. The growth factors may be used either individually or in combination of two or more kinds thereof. Especially, as concretely demonstrated in the following Examples, growth of mesenchymal stem cells can be sufficiently attained by using a medium containing no other growth factors besides the two factors, i.e., a platelet-derived growth factor (PDGF) and a basic fibroblast growth factor (bFGF), and threfore, in cases of culturing mesenchymal stem cells, only these two growth factors may be used sufficiently as growth factors to be contained in the medium.

The efficient serum-free medium may further contain a ligand (PDGF) for a platelet-derived growth factor receptor (PDGFR), and especially, a medium for culturing mesenchymal stem cells preferably contains this. PDGFR is a kind of tyrosine kinase-related receptors, and by using a medium containing a ligand for this, mesenchymal stem cells can be grown efficiently. Examples of ligands for PDGFR include PDGF-AA, PDGF-AB, PDGF-BB, PDGF-CC and PDGF-DD, all of which are well-known. The ligands for PDGFR may be used either individually or in combination of two or more kinds thereof.

The efficient serum-free medium may contain a ligand (FGF) for a basic fibroblast growth factor receptor (FGFR), and especially, the medium preferably contains this when mesenchymal stem cells are cultured. FGFR is known to mainly exist in mesenchymal cells, and by using a medium containing a ligand for this, the life time of a mesenchymal stem cell can be improved. A total of not less than 20 types of ligands for PDGFR are known to exist, which ligands include basic fibroblast growth factors (bFGFs) and acidic fibroblast growth factors (aFGFs). Examples thereof include FGF-1 and FGF-4. Especially, bFGF is known to be strongly involved in formation of tissues. All of these are well-known.

The concentration of the growth factor (in cases where a plurality of the growth factors are contained, the total concentration thereof) is preferably 0.1 to 100 ng/mL, more preferably 1 to 10 ng/mL.

The efficient serum-free medium may further contain a surfactant. It is considered that, by a medium containing a surfactant at a low concentration, there is an effect to reduce an adverse effect on cell membrane. On the other hand, it is known that addition of a surfactant at a high concentration to a medium induces cell growth inhibition and cell death. As the surfactant, nonionic surfactants such as polyoxyethylenesorbitan fatty acid ester (trade names: Tween 20, Tween 40, Tween 60, Tween 80 and the like), alkylphenoxy polyethylene glycol (trade names: Triton X-100 and the like), alkylphenyl polyethylene glycol (trade names: Triton X-114, NP-40 and the like) are preferred. The surfactants may be used either individually or in combination of two or more kinds thereof.

The concentration of the surfactant (in cases where a plurality of the surfactants are contained, the total concentration thereof) is usually 0.1 to 100 ng/mL, preferably 1 to 10 ng/mL.

The efficient serum-free medium may be the same as a known medium for mammalian cells except that it contains the above-described Edg ligand and serotonin ligand, and preferably further one or more of the above-described antioxidants, animal serum albumins, growth factors and surfactants. Therefore, basically, the efficient serum-free medium can be obtained by addition of the above-described two essential components and, preferably, further one or more of the above-described preferred components to a known basal medium. Examples of the known basal medium are as described above. Examples of the optional additive are also as described above, and one or a plurality of the above-described additives may be contained in the medium. Similarly to known basal media, the efficient serum-free medium may be used as a differentiation-inducing medium by adding thereto a differentiation inducer(s). Although examples of the differentiation inducer are described above, an inducer(s) which may be used in the present invention is(are) not restricted thereto, and may be appropriately selected from known differentiation inducers depending on the kind of the cells to be prepared through induction of differentiation.

Cell culturing itself can be carried out in the same manner as the conventional methods, and is usually carried out at 30-37°C under 5% CO₂ and 5-21% O₂. The culturing time required for differentiation induction may be appropriately selected, depending on the kind of the differentiation inducer(s) and/or cells used, as well as on the observation of the condition of the cultured cells.

Extraction of the nucleic acids in a culture medium can be carried out by the well-known, conventional method. Various reagents and kits for extraction of nucleic acids from a liquid sample are commercially available. The extraction can be easily carried out using such products.

The method *per se* for measuring nucleic acid is well known. Examples of the measurement method based on nucleic acid amplification using primers include real-time PCR and NASBA. Examples of the measurement method using probes include Northern blot, Southern blot, and array analysis using immobilized probes. Any of the well-known measurement methods may be used in the method of the present invention. Specifically, for example, the presence and the amount of the miRNA can be determined by introducing a primer site into the 3'-end of the RNA sample extracted from cultured cells to prepare cDNA and then performing real-time PCR using miRNA-specific primers, as described in the following Examples. As primers specific for miRNAs composed of the base sequences shown in SEQ ID NOs: 1-19, for example, primers composed of the base sequences shown in SEQ ID NOs: 22-40 may be used. "TaqMan Probe microRNA Assays", the commercially available kit developed by Applied Biosystems, may also be used. This kit handles various known miRNAs and makes it possible to measure the desired miRNA by real-time PCR. It is noted here that, in the present invention, the term "measurement" includes detection, quantification and semi-quantification.

In cases where a nucleic acid(s) whose release into a culture medium is initiated on a cellular change is(are) measured, the measurement may be carried out by measuring the nucleic acid(s) in aliquots of the culture medium collected with time to examine whether the nucleic acid(s) is(are) detected or not. In cases where a change in the amount of the nucleic acid(s) is measured, the measurement of the change may be carried out by, for example, taking the start of the culturing as the base point in time and taking the amount of the nucleic acid(s) at the base point in time as 1, relatively evaluating the amount of the nucleic acid(s) in the culture medium after the base point in time. The time point and the amount of the nucleic acid(s) which are taken as standards for the relative evaluation may be appropriately selected. In the evaluation, the amount of the nucleic acid(s) may be corrected by an internal standard (e.g. miR-16). For example, the evaluation of stem cells and tissue cells derived from the stem cells can be attained by analyzing the amount, pattern, etc. of the nucleic acid(s) released from the stem cells and the tissue cells derived from the stem cells. The evaluation of the effect and toxicity of a drug can be attained by analyzing the amount, pattern, etc. of the nucleic acid(s) released from cells treated with e.g. various amounts of the drug. The evaluation of the malignancy of cancer cells can be attained by analyzing the amount, pattern, etc. of the nucleic acid(s) released from cancer cells showing different malignancy. The evaluation of the presence of cancer cells can be attained by analyzing the amount, pattern, etc. of the nucleic acid(s) released from cancer cells and normal tissue cells.

### EXAMPLES

The present invention will now be described more concretely by way of an example thereof. However, the present invention is not restricted to the example below.

### Example 1: Preparation of MicroRNA Samples and Analysis Thereof-1

Human bone marrow-derived mesenchymal stem cells (produced by LONZA) were seeded in a culture vessel of 12-well culture plate at a cell density of 20000 cells/cm², and cultured in a serum-free maintenance medium, osteogenic differentiation medium or adipogenic differentiation medium for 21 days, thereby inducing differentiation into osteocytes or adipocytes. The composition of each medium used was as follows.

### Maintenance medium:

A serum-free medium prepared by adding additives for cell maintenance (final concentration: 5µM LPA (produced by Cayman), 10 µM serotonin (produced by SIGMA), 1 nM PDGF (produced by Wako Pure Chemicals), and 2.5 nM bFGF (produced by Wako Pure Chemicals)) to MEM-Alpha (produced by Wako Pure Chemicals).

### Osteogenic differentiation medium:

A serum-free medium prepared by adding osteogenic inducers (final concentration: 10 mM β-glycerophosphoric acid (produced by SIGMA), 100 nM dexamethasone, 1 mM vitamin C (produced by Wako Pure Chemicals), and 5 µM LPA (produced by Cayman)) to DMEM(produced by Wako Pure Chemicals).

### Adipogenic differentiation medium:

A serum-free medium prepared by adding adipogenic inducers (final concentration: 1 µM dexamethasone, 1 µM rosiglitazone, 500 µM isobutylmethylxanthine, 1 µM insulin, 5 µM LPA (produced by Cayman)) to DMEM (produced by Wako Pure Chemicals).

Each culture medium used for culturing for 7 days from Day 14 to Day 21 from the beginning of cell culture was collected, and floating cells were removed by filtration through a 0.22 µm filter to obtain samples.

To 250 µL of each of the samples, 750 µL of TriZOL LS reagent (produced by Invitrogen) was added and mixed, and 200 µL of chloroform was added thereto, followed by vigorous stirring and centrifugation at 12000 x g to recover the supernatants. To each supernatant, 500 µL of isopropanol was added, and the mixtures were left to stand at -30°C for 12 hours or more, followed by centrifugation at 12000 x g and then discarding the supernatants. 500 µL of 75% ethanol solution was added thereto, and the mixtures were centrifuged at 12000 x g, followed by discarding the supernatants, thereby recovering nucleic acid samples.

The nucleic acid samples were dissolved in 14 µL of H₂O, and Poly(A) Tailing was added to miRNA molecules (Poly(A)miRNA) using Poly(A) Tailing Kit (produced by Ambion).

To the Poly(A)miRNA solutions, 100 µL of H₂O was added, and thereafter 375 µL of TriZOL LS reagent (produced by Invitrogen) was added thereto, followed by adding thereto 100 µL of chloroform. The mixtures were vigorously stirred and centrifuged at 12000 x g to recover the supernatants. To each supernatant, 500 µL of isopropanol was added, and the mixtures were left to stand at -30°C for 12 hours or more, followed by centrifugation at 12000 x g and then discarding the supernatants. 500 µL of 75% ethanol solution was added thereto, and the mixtures were centrifuged at 12000 x g, followed by discarding the supernatants to recover Poly(A)miRNA samples.

The Poly(A)miRNA samples were dissolved in 10 µL of H₂O. RNA was reverse-transcribed into DNA using RTprimer (5'-GCGAGCACAGAATTAATAC GACTCACTATAGGTTTTTTTTTTTTVN-3', SEQ ID NO: 20) and Super Script II reverse transcriptase (produced by Invitrogen), and 25 µL of H₂O was added thereto to obtain cDNA samples.

Real-time PCR was carried out using Platinum SYBR Green (produced by Invitrogen) to measure hsa-miR-145, hsa-miR-24 and hsa-miR-16 contained in each cDNA sample. The primers used were as follows.
hsa-miR-145 (SEQ ID NO: 1): forward (CCGCGTCCAGTTTTCCCAGGAA, SEQ ID NO: 22)
hsa-miR-24 (SEQ ID NO: 2): forward (CCGCTGGCTCAGTTCAGCAG, SEQ ID NO: 23)
hsa-miR-16 (SEQ ID NO: 3): forward (CGCGCTAGCAGCACGTAAAT, SEQ ID NO: 24)
Consensus reverse (GCGAGCACAGAATTAATACGAC, SEQ ID NO: 21)

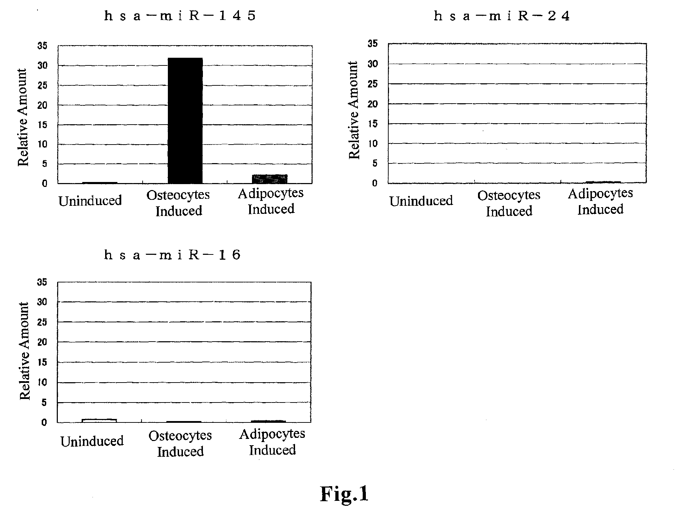

The results are shown in Fig. 1, by which the presence of miRNA released out of the cultured cells was confirmed. It was also confirmed that the pattern of the amount of miRNA released from the cultured cells varied depending on the kind of the tissue cells. For example, the amount of released hsa-miR-145 greatly increased in the case where mesenchymal stem cells differentiated into osteocytes, suggesting that the pattern of the amount of miRNA released from cultured cells varies depending on changes in the cultured cells (differentiation of stem cells); i.e., suggesting that to what degree the induction of differentiation of mesenchymal stem cells proceeds can be predicted by analyzing miRNA released therefrom. Differentiation into osteocytes and adipocytes was confirmed by Alizarin Red S staining and Oil Red O staining, respectively.

### Example 2: Preparation of MicroRNA Samples and Analysis Thereof-2

Human liver cancer-derived cell line (HuH-7, purchased from ATCC) was seeded in a culture vessel of 12-well culture plate at a cell density of 20000 cells/cm², and cells were cultured for 7 days in a serum-free maintenance medium containing carbon tetrachloride, which was known to injure liver tissue and liver cells, at a relatively low concentration of 0-1000 µM used in World J Gastroenterol. 2008 Jun 21;14(23):3693-709. The maintenance medium used herein was the same as one used in Example 1.

Each culture medium was collected after 7-day culturing, and cells were removed by filtration through a 0.22 µm filter to obtain samples.

cDNA samples of Poly(A)miRNA samples were obtained in the same manner as in Example 1.

Real-time PCR was carried out using Platinum SYBR Green (produced by Invitrogen) to measure hsa-miR-21, hsa-miR-122, hsa-miR-451 and hsa-miR-16 contained in the cDNA samples. The primers used were as follows.
hsa-miR-21 (SEQ ID NO: 4): forward (GCCCGCTAGCTTATCAGACTGATG, SEQ ID NO: 25)
hsa-miR-122 (SEQ ID NO: 5): forward (GCGCTGGAGTGTGACAATGGT, SEQ ID NO: 26)
hsa-miR-451 (SEQ ID NO: 6): forward (GCCGCAAACCGTTACCATTACT, SEQ ID NO: 27)
hsa-miR-16 (SEQ ID NO: 3): forward (CGCGCTAGCAGCACGTAAAT, SEQ ID NO: 24)
Consensus reverse (GCGAGCACAGAATTAATACGAC, SEQ ID NO: 21)

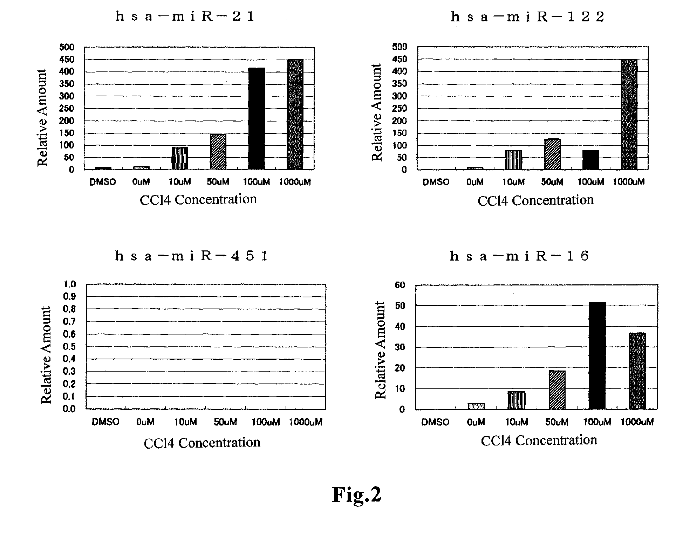

The results were shown in Fig. 2, by which the presence of miRNA released out of cultured cells which were different from stem cells was also confirmed. In addition, it was confirmed that miRNA released from cells increased depending on injury of cultured cells. These results suggest that the pattern of the amount of miRNA released into a culture medium varies depending on changes in the cultured cells, e.g., drug treatment. That means, the effect and/or the toxicity of drugs etc. can be evaluated by analyzing the amount of miRNA released into a culture medium.

### Example 3: Preparation of MicroRNA Samples and Analysis Thereof-3

Human colon cancer-derived cell line and its lymph node metastasis-derived cell line (SW480 and SW620, purchased from ATCC) were seeded in a culture vessel of 6-well culture plate at a cell density of 20000 cells/cm², and cells were cultured in a serum-free maintenance medium for 3 days. The maintenance medium used herein was the same as one used in Example 1.

Each culture medium was collected after 3-day culturing and centrifuged to obtain a supernatant, and each supernatant was filtered through a 0.22 µm filter to remove cells, thereby obtaining samples.

cDNA samples of Poly(A)miRNA samples were obtained in the same manner as in Example 1.

Real-time PCR was carried out using Platinum SYBR Green (produced by Invitrogen) to measure hsa-miR-20a, hsa-miR-892a, hsa-miR-22*, hsa-miR-19a, hsa-miR-484, hsa-miR-638, hsa-miR-125b, hsa-miR-339-5p, hsa-miR-532-3p, hsa-miR-142-3p, hsa-miR-138, hsa-miR-186, hsa-miR-223 contained in the cDNA samples. The primers used were as follows.
hsa-miR-20a (SEQ ID NO: 7): forward (CCGCCGCTAAAGTGCTTATAGTG, SEQ ID NO: 28)
hsa-miR-892a (SEQ ID NO: 8): forward (CCGCCACTGTGTCCTTTCTGC, SEQ ID NO: 29) hsa-miR-22* (SEQ ID NO: 9): forward (CCGCGAGTTCTTCAGTGGCAA, SEQ ID NO: 30)
hsa-miR-19a (SEQ ID NO: 10): forward (CCGCCTGTGCAAATCTATGCA, SEQ ID NO: 31) hsa-miR-484 (SEQ ID NO: 11): forward (CCGTCAGGCTCAGTCCCCT, SEQ ID NO: 32)
hsa-miR-638 (SEQ ID NO: 12): forward (CCGCAGGGATCGCGGGC, SEQ ID NO: 33)
hsa-miR-125b (SEQ ID NO: 13): forward (CCGCGTCCCTGAGACCCTAA, SEQ ID NO: 34)
hsa-miR-339-5p (SEQ ID NO: 14): forward (CCGTCCCTGTCCTCCAGGA, SEQ ID NO: 35)
hsa-miR-532-3p (SEQ ID NO: 15): forward (CCCTCCCACACCCAAGGCT, SEQ ID NO: 36)
hsa-miR-142-3p (SEQ ID NO: 16): forward (CCGCCTGTAGTGTTTCCTACTTT, SEQ ID NO: 37)
hsa-miR-138 (SEQ ID NO: 17): forward (CCGGCAGCTGGTGTTGTGAA, SEQ ID NO: 38)
hsa-miR-186 (SEQ ID NO: 18): forward (CCGCCGCAAAGAATTCTCCTTTT, SEQ ID NO: 39)
hsa-miR-223 (SEQ ID NO: 19): forward (CCGCCGTGTCAGTTTGTCAAATA, SEQ ID NO: 40)
Consensus reverse (GCGAGCACAGAATTAATACGAC, SEQ ID NO: 21)

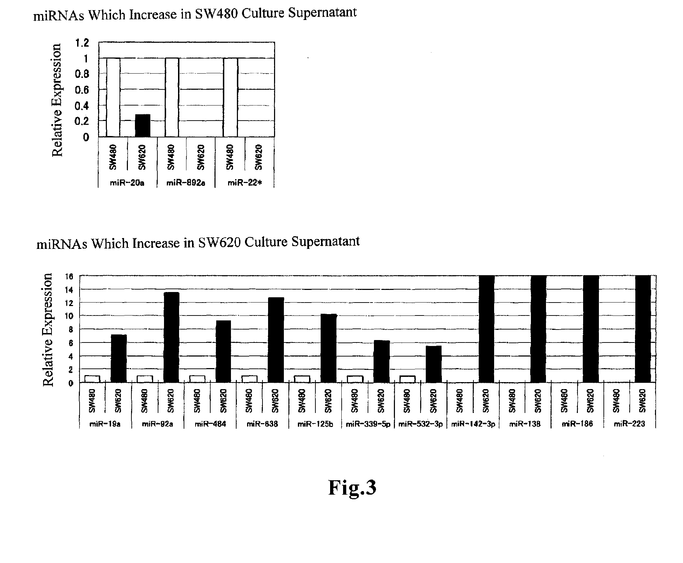

The results were shown in Fig. 3. Since SW620 is a cell line derived from a metastatic lymph node, its malignancy is higher than the original colon cancer-derived SW480 cells. By Fig. 3, it was confirmed that miRNAs released from the cells increased or decreased depending on the malignancy development of cancer cells, suggesting that the pattern of the amount of miRNAs released into the medium varies depending on the malignancy development of cancer cells. In addition, the presence of common miRNAs released from cancer cells regardless of the cancer malignancy development was confirmed, which suggests that there are miRNAs released from cancer cells in common; i.e., that evaluation of whether cancer cells are present or not can be attained.

### Example 4: Preparation of MicroRNA Samples and Analysis Thereof-4

In the same manner as in Example 1, human bone marrow-derived mesenchymal stem cells (produced by LONZA) were seeded in a culture vessel of 12-well culture plate at a cell density of 20000 cells/cm², and cultured in a serum-free maintenance medium, osteogenic differentiation medium or adipogenic differentiation medium for 21 days, thereby inducing differentiation into osteocytes or adipocytes.

Each culture medium used for culturing for 7 days from Day 14 to Day 21 from the beginning of cell culture was collected, and floating cells were removed by filtration through a 0.22 µm filter to obtain samples.

To 250 µL of each of the samples, 750 µL of Trizol LS reagent (produced by Invitrogen) was added and mixed, and 200 µL of chloroform was added thereto, followed by vigorous stirring and centrifugation at 12000 x g to recover the supernatants. To each supernatant, 500 µL of isopropanol was added, and the mixtures were left to stand at -30°C for 12 hours or more, followed by centrifugation at 12000 x g and then discarding the supernatants. 500 µL of 75% ethanol solution was added thereto, and the mixtures were centrifuged at 12000 x g, followed by discarding the supernatants, thereby recovering nucleic acid samples.

The nucleic acid samples were dissolved in 14 µL of H₂O, and cDNA samples were prepared using TaqMan (registered trademark) MicroRNA RTprimer (produced by Applied Biosystems).

Real-time PCR was carried out using TaqMan (registered trademark) MicroRNA Realtime primers (produced by Applied Biosystems) to quantitatively measure the cDNA samples, and the measured values were corrected by hsa-miR-16. As for the primers, TaqMan (registered trademark) MicroRNA ASSAY Probes (produced by Applied Biosystems) commercially available under the following ASSAY IDs were used.
hsa-miR-130a (SEQ ID NO: 41): 000454 (ASSAY ID)
hsa-miR-143 (SEQ ID NO: 42): 002249 (ASSAY ID)
hsa-miR-214 (SEQ ID NO: 43): 002306 (ASSAY ID)
hsa-miR-365 (SEQ ID NO: 44): 001020 (ASSAY ID)
hsa-miR-16 (SEQ ID NO: 3): 00391 (ASSAY ID)

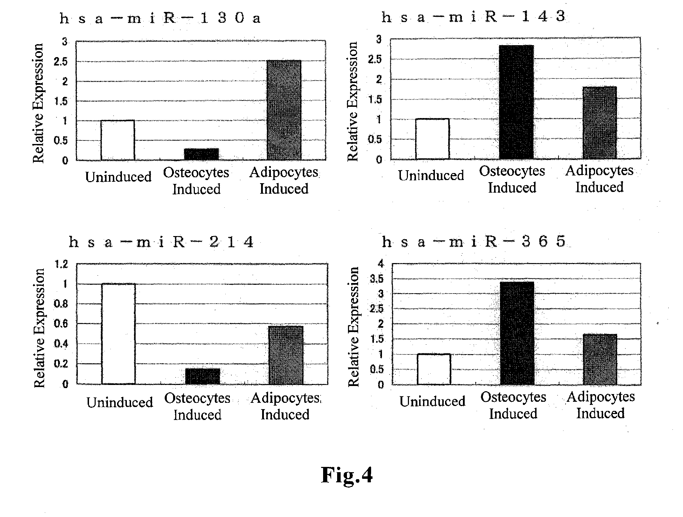

The results were shown in Fig. 4, by which it was confirmed that miRNAs released from mesenchymal stem cells into the outside of the cells varied by differentiation into tissue cells including adipocytes and osteocytes. These results suggest that the quality of mesenchymal stem cells and tissue cells can be evaluated by analyzing released miRNAs. The followings are marker candidates for each tissue cells when mesenchymal stem cells are induced to differentiate into them.
Undifferentiated cells (uninduced): hsa-miR-214
Tissue cells (osteocytes and adipocytes): hsa-miR-143
Osteocytes: hsa-miR-365
Adipocytes: hsa-miR-130a

### Additional Statements of Invention

1. A method for evaluating cultured cells, comprising culturing cells in a serum-free medium, and measuring at least one nucleic acid released from the cells into the culture medium.
2. The method according to 1, wherein said nucleic acid is microRNA.
3. The method according to 2, wherein at least one of the microRNAs composed of the base sequences shown in SEQ ID NOs: 1, 3-5, 7-19, 41-44 in SEQUENCE LISTING is measured.
4. The method according to 3, wherein at least one of the microRNAs composed of the base sequences shown in SEQ ID NOs: 1, 3-5, 7-19 in SEQUENCE LISTING is measured.
5. The method according to any one of 1 to 4, wherein said cells are originated from a mammal, and wherein said serum-free medium contains a ligand for an endothelial cell differentiation gene (Edg) family receptor and a ligand for a serotonin receptor.
6. The method according to 5, wherein said ligand for an endothelial cell differentiation gene family receptor is at least one selected from the group consisting of: lysophosphatidic acid (LPA) and salts thereof; sphingosine-1-phosphate (S1P); and agonists of endothelial cell differentiation gene (Edg) family receptors.
7. The method according to 5 or 6, wherein said ligand for a serotonin receptor is at least one selected from the group consisting of: serotonin and salts thereof; and agonists of serotonin receptors.
8. The method according to any one of 1 to 7, which is a method for evaluating differentiation of stem cells.
9. The method according to 8, wherein differentiation of stem cells is evaluated using at least one of the microRNAs composed of the base sequences shown in SEQ ID NOs: 1, 41-44 as an indicator.
10. The method according to 9, wherein differentiation into osteocytes is evaluated using a microRNA composed of the base sequence shown in SEQ ID NO: 1 or 44 as an indicator.
11. The method according to 10, wherein differentiation into osteocytes is evaluated using a microRNA composed of the base sequence shown in SEQ ID NO: 1 as an indicator.
12. The method according to 9, wherein differentiation into adipocytes is evaluated using a microRNA composed of the base sequence shown in SEQ ID NO: 41 as an indicator.
13. The method according to 9, wherein differentiation into tissue cells is evaluated using a microRNA composed of the base sequence shown in SEQ ID NO: 42 as an indicator.
14. The method according to 9, wherein whether differentiation of stem cells occurs is evaluated using a microRNA composed of the base sequence shown in SEQ ID NO: 43 as an indicator.
15. The method according to any one of 1 to 7, which is a method for evaluating cell injury.
16. The method according to 15, wherein injury to the cultured cells is evaluated using at least one of the microRNAs composed of the base sequences shown in SEQ ID NOs: 3-5 as an indicator.
17. The method according to 16, wherein said cultured cells are liver cells.
18. The method according to any one of 1 to 7, which is a method for evaluating effect, influence, toxicity, etc. of a chemical substance, biological material, environmental stimulus, etc. against cultured cells.
19. The method according to any one of 1 to 7, which is a method for evaluating the presence of cancer cells.
20. The method according to any one of 1 to 7, which is a method for evaluating malignancy of cancer cells.
21. The method according to 19 or 20, wherein at least one of the microRNAs composed of the base sequences shown in SEQ ID NOs: 7-19 is used as an indicator.
22. The method according to 21, wherein said cancer cells are colon cancer cells.
23. A method for screening a biomarker(s), comprising culturing cells in a serum-free medium, and measuring a nucleic acid(s) released from the cells into the culture medium.
24. The method according to 23, wherein at least one of the microRNAs composed of the base sequences shown in SEQ ID NOs: 1, 3-5, 7-19, 41-44 in SEQUENCE LISTING is measured.
25. The method according to 24, wherein at least one of the microRNAs composed of the base sequences shown in SEQ ID NOs: 1, 3-5, 7-19 in SEQUENCE LISTING is measured.

## Claims

1. A method for evaluating cultured cells, comprising culturing cells in a serum-free medium, and measuring at least one nucleic acid released from the cells into the culture medium, wherein said nucleic acid is microRNA, and wherein at least one of the microRNAs composed of the base sequences shown in SEQ ID NOs: 17, 19, 7-16, and 18 in the SEQUENCE LISTING is measured.

2. The method according to claim 1, wherein said cells are originated from a mammal, and wherein said serum-free medium contains a ligand for an endothelial cell differentiation gene (Edg) family receptor and a ligand for a serotonin receptor.

3. The method according to claim 2, wherein said ligand for an endothelial cell differentiation gene family receptor is at least one selected from the group consisting of:
lysophosphatidic acid (LPA) and salts thereof; sphingosine-1-phosphate (SIP); and agonists of endothelial cell differentiation gene (Edg) family receptors, and/or wherein said ligand for a serotonin receptor is at least one selected from the group consisting of: serotonin and salts thereof; and agonists of serotonin receptors.

4. The method according to any one of claims 1 to 3, which is a method for evaluating the presence of cancer cells

5. The method according to any one of claims 1 to 3, which is a method for evaluating malignancy of cancer cells.

6. The method according to claim 4 or 5, wherein at least one of the microRNAs composed of the base sequences shown in SEQ ID NOs: 17, 19, 7-16, and 18 is used as an indicator.

7. The method according to claim 6, wherein said cancer cells are colon cancer cells.

8. A method for screening a biomarker(s), comprising culturing cells in a serum-free medium, and measuring a nucleic acid(s) released from the cells into the culture medium, wherein at least one of the microRNAs composed of the base sequences shown in SEQ ID NOs: 17, 19, 7-16, and 18 in the SEQUENCE LISTING is measured.
